(19) Europäisches Patentamt
European Patent Office
Office européen des brevets

(11) EP 1 369 478 A1

(12) # EUROPEAN PATENT APPLICATION
published in accordance with Art. 158(3) EPC

(43) Date of publication:
**10.12.2003 Bulletin 2003/50**

(21) Application number: **02712399.1**

(22) Date of filing: **15.02.2002**

(51) Int Cl.[7]: **C12N 15/09**, C07K 14/705, C07K 16/28, C12N 5/10, C12Q 1/02, C12Q 1/68, C12P 21/08, G01N 33/15, G01N 33/50

(86) International application number:
**PCT/JP02/01320**

(87) International publication number:
**WO 02/064770 (22.08.2002 Gazette 2002/34)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **15.02.2001 JP 2001038378**

(71) Applicant: **MOCHIDA PHARMACEUTICAL CO., LTD.**
**Shinjuku-ku Tokyo 160-8515 (JP)**

(72) Inventors:
- **NAKAMURA, Yusuke**
  **Yokohama-shi, Kanagawa 225-0011 (JP)**
- **SUGANO, Sumio**
  **Suginami-ku, Tokyo 167-0052 (JP)**
- **KAWANO, Hiroyuki,**
  **c/o MOCHIDA PHARMA. CO., Ltd.**
  **Tokyo 160-8515 (JP)**

(74) Representative: **Wablat, Wolfgang, Dr.Dr.**
**Patentanwalt,**
**Potsdamer Chaussee 48**
**14129 Berlin (DE)**

(54) **NOVEL SCAVENGER RECEPTOR CLASS A PROTEIN**

(57) A gene and a protein participating in arteriosclerosis caused by foam macrophages and a method of efficiently evaluating an agent controlling an activity of this protein are provided.

A DNA comprising the nucleotide sequence represented by SEQ ID NO: 1; a scavenger reporter-like protein encoded by this DNA; an antisense nucleic acid to this DNA sequence; and a method of evaluating an agent capable of controlling an activity of this protein are also provided.

EP 1 369 478 A1

## Description

### FIELD OF THE INVENTION

The present invention relates to a novel scavenger receptor class A subtype and its fragment, a DNA encoding said protein or its fragment, an expression vector including said DNA, a transformant produced by transformation with said expression vector, and an antibody having a reactivity with said protein or its fragment.

### BACKGROUND OF THE INVENTION

Arteriosclerosis developed with hyperlipidemia is characterized by the deposition of foamed macrophages in which a large amount of cholesterol is accumulated into atherosclerotic lesions. The origin of the foam cells is macrophages from monocytes in peripheral blood and vascular smooth muscle cells, but in most of initial lesions it is macrophages. It is believed that in atherosclerosis, vascular endothelial cells are first damaged. Many factors participates in the damage, among which LDL and its modification are important. LDL in blood infiltrates into subendothelium and then subjected to various modifications to thereby turn into a modified LDL. On endothelial cells damaged by causes including the effect of modified LDL, an adhesion molecule expresses. Monocytes in peripheral blood are adhered via the adhesion molecule so that they infiltrate into subendothelium. The thus-infiltrated monocytes are differentiated into macrophages, which uptake a modified LDL accumulated below an endothelium to turn into foam cells. By these foam cells, an initial lesion of atherosclerosis is formed. If this lesion is further progressed, the foam cells in which excess cholesterol ester is accumulated are dead to form lipid cores which may cause cardiovascular events. . A scavenger receptor (SR) is a receptor protein binding to or incorporating a modified LDL which is one of the causes of foam cell formation.

Many SR cDNAs have been cloned from various organisms including bovine (Kodama et al., Nature, Vol. 343, pp. 531-535 (1990)) and human (Matsumoto et al., Proc. Natl. Acad. Sci. USA, Vol. 87, pp. 9133-9137 (1990)). As the result, it was clarified that SR is mainly classified into class A and class B and some subtype proteins are present in each class.

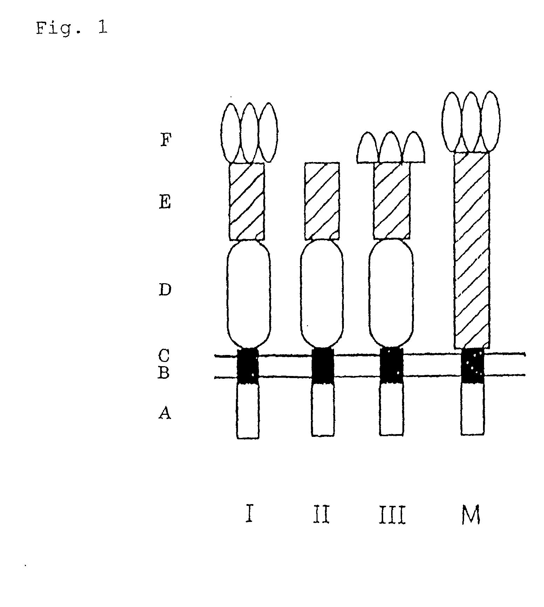

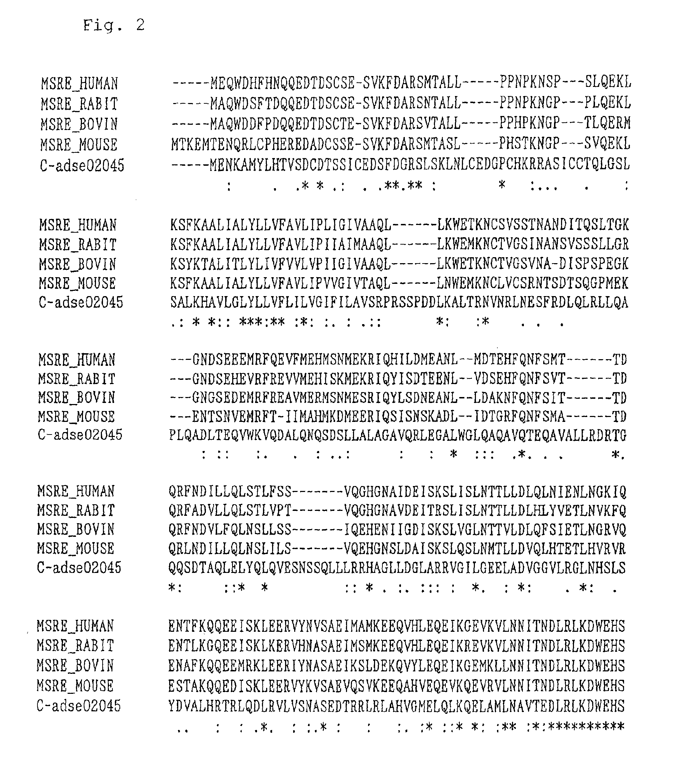

It has been reported that a macrophage scavenger receptor (MSR) belongs to class A and this class A has four subtypes, i.e. I, II, III and MARCO subtypes. These subtypes are trimer membrane glycoproteins of inside-out type. Especially the subtypes I to III are characterized by that each of these subtypes comprises six domains, specifically from its N-terminal a cytoplasmic domain, a transmembrane domain, a spacer domain, an α-helicalcoiled-coil domain, a collagen like domain and a C-terminal specific domain (see Fig. 1).

A cytoplasmic domain is characterized in that it has a characteristic tight turn structure which is observed in an endocytosis signal similar to NPXY sequence found in an LDL receptor and an insulin receptor and YXRF sequence found in a transferin receptor. It is thought that in fact the endocytosis is inhibited by deleting these sequences.

An α-helicalcoiled-coil domain is characterized in that an active receptor is formed by associating three scavenger receptor polypeptides as protein primary structures. This domain takes a dextral hepted repeat rotating twice every 7 amino acids, i.e. a helicalcoiled-coil structure in which hydrophobic amino acids such as leucine and isoleucine occurring every 7 amino acids are directed inwardly and polar amino acids and sugar chain binding sites are directed outwardly so that three polypeptides form a trimer. And, it binds to ligands such as a modified LDL so as to enter into cells. Thus, it has a function of dissociating ligands by changing a high structure of a receptor with the lowering in pH in an endosome. Especially, it is experimentally proved that histidine present at the side of a collagen like domain after this domain functions in the dissociation of ligands within cells. Further, an antibody recognizing 15 amino acids region whose central is histidine of this coiled-coil domain inhibits a calcium-independent adhesion of macrophages to cells, from which it is suggested that MSR has a function as a cell adhesion molecule.

A collagen like domain is characterized in that it has a repeat structure of glycine (Gly)-X-Y inherent in collagen. Many positively charged amino acids suitable for the binding to negatively charged ligands are assembled at its C-terminal. It is confirmed that a mutant missing 22 amino acids at C-terminal has not a ligand bindability, from which it is guessed that this collagen domain is a site of MRS participating in the binding to ligands.

A C-terminal specific domain is characterized by that it is rich in cysteine and it has a high homology with a complement factor 1, CD5, CD6 and the like. Spaces on an amino acid sequence of six cysteine residues are completely conserved irrespective of animal species in a subtype of the same type, for example a subtype of type I, but the entire or a part of this domain is lost in subtypes of types II and III. Thus, the domain structure is significantly different as a whole.

MSR having the aforementioned structural characteristics incorporates excess modified LDL so that macrophages are turned into foam cells. It is pointed out that the incorporation of modified LDL and the foaming of macrophages play important roles in the development of arteriosclerosis. In fact, in a double knockout mouse in which both MSR and apoE are inactivated genetically, the reduction of atherosclerotic lesions is confirmed as compared with that of a knockout mouse in which only apoE is inactivated. It is proved from this fact that MSR significantly participates in the development of arteriosclerosis. Further, it is clear that MSR participates in the protection against bacterial infections and the incorporation of advanced glycation end products (AGE) which may cause diabetic vascular disorders (Nature, Vol. 386, pp. 292-296 (1997)).

However, a role of each subtype protein of SR class A has never been clarified. In addition, it is unknown whether SR other than the subtypes which have been already reported is present or not. If present, its functional role is also unknown.

An object of the present invention is to identify a novel protein associated with arteriosclerosis which is distinguishable from a known MSR and its gene and thereby to provide amedicine and a method useful for preventing and treating arteriosclerotic diseases.

## SUMMARY OF THE INVENTION

The present invention relates to a novel gene (hereinafter referred to "adse"), a SR-like protein encoded by said gene, especially a novel subtype ADSE (hereinafter referred to "ADSE") guessed to belong to class A. Thepresent invention also provides a host cell transformed with said gene and a method of producing a recombinant ADSE using said transformant.

<nucleic acid>

The present invention provides a gene adse encoding ADSE as described below in more detail. Specifically, the gene adse means a DNA encoding a scavenger receptor-like protein comprising the amino acid sequence represented by SEQ ID NO:2. It includes a cDNA of SEQ ID NO:1 and 3 as well as a genomic DNA that said cDNA comes from. Although this gene can be isolated and identified from a human adipose tissue, the gene may be a DNA obtained by cloning using a genetic engineering technique such as a hybridization or a chemical synthetic technique such as a phosphoramidite method based on the sequence as disclosed herein. The form of the gene may be a cDNA, a genomic DNA and a chemically synthesized DNA, however not limited thereto.

The DNA of the present invention may be a single strand DNA. Alternatively, it may bind to a DNA or an RNA having the sequence complementary thereto to form a double-or triple-strand. The DNA may be labeled with an enzyme such as horseradish peroxidase (HRPO); a radioactive isotope; a fluorescent substance; a chemiluminescent substance; and the like.

If the nucleotide sequence of adse is provided, a sequence of an RNA and a sequence of a complementary DNA and RNA are univocally determined. Therefore, it should be understood that the present invention also provides an RNA corresponding to the DNA of the present invention as well as a DNA and an RNA having a sequence complementary to the DNA of the present invention. "DNA" and "polynucleotide" are interchangeably used herein.

The DNA of the present invention also includes a DNA hybridizable with the DNA comprising the nucleotide sequence represented by SEQ ID No. 1 under stringent conditions.

Variations of the nucleotide sequence represented by SEQ ID NO:1 are acceptable as long as they are hybridizable with the DNA comprising the nucleotide sequence represented by SEQ ID NO:1 under stringent conditions and a protein encoded by said DNA is a scavenger receptor-like protein. It should be understood that a DNA sequence partially modified by, for example, the presence of plural codons encoding the same amino acid residue due to the degeneracy of codon; and various artificial treatments such as site-specific mutation, random mutation by treating with a mutagen, mutation, deletion, linkage and the like of the DNA fragment by cleaving with a restriction enzyme are included within the present invention as long as it is hybridizable with the DNA represented by SEQ ID No. 1 under stringent conditions and encodes a scavenger receptor-like protein even if its sequence is different from the DNA sequence represented by SEQ ID No. 1.

The DNA mutant is acceptable as long as it has a homology with the DNA sequence represented by SEQ ID No. 1 of at least 70%, preferably at least 80%, more preferably at least 90%. The homology in DNA sequence can be analyzed by BLAST (J. Mol. Evol., Vol. 36, pp. 290-300 (1993) ; J. Mol. Biol., Vol. 215, pp. 403-410 (1990)). The term "hybridizable" means that a DNA is hybridizable with the nucleic acid represented by SEQ ID No. 1 by southern hybridization under stringent conditions. For example, if a probe labeled with DIG Labeling kit (Cat No. 1175033 of Rosche Diagnostics) is used, the hybridization is conducted in a DIG Easy Hyb solution (Cat No. 1603558 of Rosche Diagnostics) at the temperature of, for example, 32°C (preferably 37°C, more preferably 42°C) and the membrane is washed in, for example, a 0.5 x SSC solution (containing 0.1% (w/w) SDS) at 50°C (preferably 65°C) (note: 1 x SSC is 0.15M NaCl and 0.015M sodium citrate).

The DNA comprising the nucleotide sequence represented by SEQ ID NO:1 or its partial fragment is believed to be useful as a specific probe for diseases in which the protein of the present invention participates such as arteriosclerosis.

The DNA of the present invention can be used to commercially produce ADSE. And, the DNA can be used for testing the expression status of the protein of the present invention in a tissue by labeling with an enzyme or the like. That is, an expression amount of mRNA as an index of an expression amount of the protein of the present invention in a cell is confirmed by using the DNA as a probe so that a cell and culturing conditions of the cell suitable for the preparation of the protein of the present invention can be determined. In addition, diseases in which the protein of the present invention participates such as arteriosclerosis can be diagnosed.

Further, an abnormality or polymorphism on the nucleic acid sequence can be tested and/or diagnosed by any method such as PCR-RFLP (Restriction fragment length polymorphism) method, PCR-SSCP (Single strand conformation polymorphism) method, sequencing method and the like, using a part of the DNA of the present invention as a primer.

And, the DNA of the present invention can be used in gene therapy for preventing the development of diseases such as arteriosclerosis and the like in order to lower the concentration of a modified LDL in blood, by introducing the DNA of the present invention into *in vivo* cells and imparting an activity of incorporating modified LDL to the cells.

The DNA of the present invention is very useful in the preparation of a transformant, the production of a recombinant protein ADSE using said transformant and the screening of a compound specifically inhibiting the expression of ADSE.

The transformant of the present invention can be produced according to a method known for those skilled in the art. For example, the DNA of the present invention can be incorporated into a suitable host cell using any one of vectors commercially available or easily obtained by those skilled in the art. Then, the expression of the gene adse within the host cell canbe suitably controlled by placing the gene adse under the influence of an expression control gene, typical examples of which are a promoter and an enhancer. This technique is suitable for being used in the production of ADSE using the transformed host cell as well as the investigation of mechanisms how to regulate the expression of the gene adse and the screening of an agent capable of controlling the expression of said gene.

For example, by contacting any test substances with a cell transformed with the vector including the gene adse under suitable conditions, an agent capable of enhancing or inhibiting the expression of the gene adse can be searched among the test substances or evaluated.

By using the DNA of the present invention in combination with a known technique, a transgenic animal can be produced from a suitable animal such as mouse or the like. The transgenic animal is also very useful as a arteriosclerotic model animal since atherosclerotic lesions characteristic in arteriosclerosis can be caused in the animal by giving a high cholesterol food thereto. Thus, it is expected to significantly accelerate the development of effective drugs for arteriosclerosis caused by ADSE by using the above animal model.

And, it is possible to produce the so-called knockout non-human animal in which a gene corresponding to the human adse is destroyed in the animal by using the gene adse of the present invention. By analyzing physical, biological, pathological and genetic characteristic of this model animal, functions of the gene and the protein of the present invention can be elucidated. Further, by introducing the human adse of the present invention into the animal whose endogenous gene is destroyed, a model animal having only human adse can be produced. This model animal is useful in development and evaluation of a drug targeting the human adse introduced in said model.

<protein ADSE>

The protein ADSE encoded by adse is a scavenger receptor-like protein comprising the amino acid sequence represented by SEQ ID NO:2. Especially, it is judged that the protein ADSE is a novel subtype protein belonging to class A, from structural characteristics found in its amino acid sequence.

ADSE contains characteristic structural domains present in the subtype protein belonging to class A, especially subtype I, specifically all of six domains from the N-terminal, i.e. a cytoplasmic domain (1 to 59 a.a.), a transmembrane domain (60 to 91 a.a.), a spacer domain (92 to 126 a.a.), an $\alpha$-helicalcoiled-coil domain (127 to 305 a.a.), a collagen like domain (306 to 384 a.a.) and a C-terminal specific domain (385 to 495 a.a.). Therefore, it is recognized that ADSE is a trimer membrane glycoprotein of inside-out type similar to other subtype proteins belonging to SR class A. Similar to other subtype proteins belonging to SR class A, it is thought that ADSE is bound to modified LDLs such as an acetylated LDL, an malondialdehydated LDL, an oxidized LDL, an acetoacetylated LDL, a maleylated LDL, a succinylated LDL and the like, a maleylated albumin, AGE (advanced glycation end product), LPS (lypopolysaccharide) and the like as ligands. The scavenger receptor-like protein of the present invention is understood as a receptor protein having an activity of binding to the above ligands. More preferably, ADSE is a protein having an activity of binding to a ligand such as a modified LDL and the like and incorporating it in a cell.

As mentioned above, ADSE maintains sufficiently the characteristics found in the scavenger receptor, class A at a domain structural level. While, when compared with the subtype I of human SRAI showing a highest homology with respect to the entire amino acid sequence, ADSE shows a homology of about 42.6%. When compared with the cysteine-rich domain showing a highest homology with respect to each domain structure, ADSE shows a homology of only about 70%. It is strongly suggested from these facts that ADSE plays characteristic roles which are not appeared in other subtypes during the development and the progression of atherosclerosis. Accordingly, a pharmaceutical compound targeting ADSE is expected to be useful as a medicine having an unknown property.

A polypeptide or protein comprising an amino acid sequence wherein substitution, deletion and/or addition of one or more amino acids had occurred in the amino acid sequence represented by SEQ ID No. 2 are included within the scope of the present invention as long as it is a scavenger receptor-like protein.

Side chains of amino acid residues which are constitutional elements of a protein are different in terms of hydrophobicity, charge, size and the like, but they are known to have several highly conservative relationships since they do not substantially affect a three-dimensional structure (also called as configuration) of the entire protein. Examples of the substitutions of amino acid residues include glycine (Gly) and proline (Pro); Gly and alanine (Ala) or valine (Val); leucine (Leu) and isoleucine (Ile); glutamic acid (Glu) and glutamine (Gln); aspartic acid (Asp) and asparagine (Asn); cysteine (Cys) and threonine (Thr); Thr and serine (Ser) or Ala; lysine (Lys) and arginine (Arg); and the like. Since Ala, Val, Leu, Ile, Pro, methionine (Met), phenylalanine (Phe), tryptophane (Trp), Gly and Cys are classified as non-polar amino acids, they are understood to have similar properties to each other. Non-charged polar amino acids include Ser, Thr, tyrosine (Tyr), Asn and Gln. Acidic amino acids include Asp and Glu. Basic amino acids include Lys, Arg and histidine (His). Even if the conservation as defined above is lost, many mutants maintaining functions essential for the protein (in the present invention, the function as a scavenger receptor-like protein) are known for those skilled in the art. Further, in several similar proteins conserved between different species, it is recognized that they maintain essential functions even if several amino acids are deleted or inserted concentratedly or scatteringly.

Accordingly, a mutant protein resulting from substitution, insertion and/or deletion of one or more amino acids in the amino acid sequence represented by SEQ ID No. 2 are included within the scope of the present invention as long as it is a scavenger receptor-like protein.

The above changes in amino acids are found in the nature such as a mutation caused by a gene polymorphism or the like. Further, it can be produced artificially according to a known method for those skilled in the art, for example, mutagenesis using a mutagene such as NTG and site-directed mutagenesis using various recombinant gene techniques. The site and the number of the mutation of amino acids are not particularly limited as long as the resultant mutant protein is a scavenger receptor-like protein. The mutation number is generally within several tens of amino acids, preferably within 10 amino acids, more preferably within 1 or several amino acids.

In the present invention, ADSE can be understood as the entire receptor having all of the domain structures as mentioned above. ADSE can be also understood as a partial peptide maintaining characteristic domains, especially a domain involved in the ligand bindability. It has been reported that among receptor proteins, free (or solubilized) partial peptides maybe present by removing a partial fragment containing a ligand binding site from other domains while maintaining a specific configuration, and the like. Since the above partial peptide maintains a bindability to specific ligands, the search of a compound having a bindability to the above receptor becomes possible. It should be understood that a partial peptide of ADSE is a substance substantially equivalent to the present invention as long as it has a ligand bindability. Especially in ADSE, it is estimated that a ligand bindability is maintained in a partial peptide at C-terminal which contains an α-helicalcoiled-coil domain. Preferable embodiment of the partial peptide includes a peptide containing any one of a spacer domain (92 to 126 a.a.), an α-helicalcoiled-coil domain (127 to 305 a.a.), a collagen like domain (306 to 384 a.a.) and a C-terminal specific domain (385 to 495 a.a.). In view of a ligand bindability, a peptide containing at least a collagen like domain (306 to 384 a.a.) is preferable. The entire or a part of other domains may be linked or any other protein or peptide may be fused as long as at least a collagen like domain (306 to 384 a.a.) is contained.

The protein or its partial peptide of the present invention can be used in screening an agent capable of controlling an activity of said protein. The thus-searched compounds and the like are expected to be useful as an effective therapeutic or preventive agent for diseases associated with the protein of the present invention such as arteriosclerosis.

<Antibody>

Further, the present invention provides an antibody binding to ADSE. The antibody of the present invention is an antibody specifically recognizing the entire ADSE or its partial peptide as an antigen. It includes a monoclonal antibody and/or a polyclonal antibody. And, it may be an antibody belonging to any one of five classes (IgG, IgA, IgM, IgD and IgE) classified by the structure, physical-chemical properties and immunological properties of immunoglobulins or either subclass classified by the type of H chain. Further, it may be a fragment such as $F(ab')'_2$ produced by digesting an immunoglobulin with, for example, pepsin, Fab produced by digesting an immunoglobulin with papain and the like, or a chimera antibody and a humanized antibody. An antibody not only specifically recognizing ADSE or its partial peptide but also having the function of controlling an activity of ADSE is also included within the present invention. Example of an antibody having the function of controlling an activity of ADSE includes a neutralizing antibody inhibiting the binding of ADSE to ligands. These antibodies are useful in investigation or clinical detection of C-ADSE and the like.

<Antisense nucleic acid>

The present invention provides the so-called antisense nucleic acid capable of inhibiting the biosynthesis of ADSE at a nucleic acid level *in vivo.* The antisense nucleic acid means a nucleic acid which binds to DNA or RNA involved in carrying a genetic information during either of a transcription stage from a genome region to a pre-mRNA essential for the production of mRNA encoding the ADSE protein, a processing stage from the pre-mRNA to a mature mRNA, a stage of passing through a nuclear membrane or a translation stage into a protein so as to affect the normal stream of the transmission of the genetic information and thereby to inhibit the expression of the protein. It may comprises a sequence complementary to the entire nucleotide sequence of the gene adse or either part of the sequence. Preferably, it is a nucleic acid (including DNA and RNA) comprising a sequence corresponding to or complementary to the nucleotide sequence represented by SEQ ID NO: 1 or 2. When the mRNA transcripted from the genome region contains an intron structure or a untranslated region at 5' or 3'-terminal, an antisense nucleic acid corresponding to or complementary to the sequence of the untranslated region will have functions equivalent to those of the antisense nucleic acid of the present invention.

The antisense nucleic acid of the present invention includes a DNA and an RNA as well as all of derivatives similar to the DNA and the RNA in configuration and functions. The antisense nucleic acid includes a nucleic acid having any other substance bound at 3'- or 5'-terminal, a nucleic acid wherein at least one of bases, sugars and phosphates of the oligonucleotide is substituted or modified, a nucleic acid having a non-naturally occurring base, sugar or phosphate, a nucleic acid having a backbone other than the sugar-phosphate backbone and the like. These nucleic acids are suitable as derivatives, in which at least one of a nuclease resistance, a tissue selectivity, a cell permeability and a binding power is improved. That is, the form of the nucleic acid is not limited as long as the nucleic acid can inhibit the activity and the expression of ADSE.

And, the antisense nucleic acid having a nucleotide sequence complementary to a nucleotide sequence hybridizable with a loop portion of mRNA forming a stem loop, i.e. the nucleotide sequence of a region forming a stem loop is generally preferable in the present invention. Alternatively, an antisense nucleic acid capable of binding to near a translation initiation codon, a ribosome binding site, a capping site and a splicing site, i.e. an antisense nucleic acid having a sequence complementary to that of these sites is also preferable since generally it can be expected to be very effective in inhibiting the expression.

In order to make the above antisense nucleic acid introduced into a cell and act efficiently, it is preferable that the length of the antisense nucleic acid of the present invention is 15 to 30 bases, preferably 15 to 25 bases, more preferably 18 to 22 bases.

The effect of the antisense nucleic acid of the present invention in inhibiting the expression can be evaluated by a known method, for example, by preparing an expression plasmid by linking a reporter gene such as luciferase and the like to the DNA containing a part of an expression control region, a 5'-untranslated region, a regionnear a translational initiation site or a translated region of the gene of the present invention, adding a test substance in a system such as a system comprising *in vitro transcription* (Ribo max systems; Promega) combined with *in vitro* translation (Rabbit Reticulocyte Lysate Systems; Promega) under the condition where the gene of the present invention is transcripted or translated and then determining an expression amount of the reporter gene.

The antisense nucleic acid of the present invention is useful as an agent for preventing or treating diseased associated with ADSE since it can inhibit the expression of ADSE *in vivo.*

## DETAILED DESCRIPTION OF THE INVENTION

<Nucleic acid>

Example of the method for obtaining the DNA of the present invention from a DNA library includes a method comprising screening a suitable genomic DNA library or cDNA library according to a screening method such as a screening method via hybridization, an immunoscreening method using an antibody and the like, amplifying a clone having the desired DNA and cleaving the DNA with a restriction enzyme or the like. In the screening method via hybridization, the hybridization can be conducted for any cDNA library using the DNA having the nucleotide sequence represented by SEQ ID No. 1 or a part thereof labeled with $^{32}P$ or the like as a probe according to a known method (see, for example, Maniatis, T. et al., Molecular Cloning, a Laboratory Manual, Cold Spring Harbor Laboratory, New York (1982)). The antibody used in the immunoscreening method may be the antibody of the present invention as described below. The novel DNA of the present invention may be also obtained by PCR (Polymerase Chain Reaction) using a genomic DNA library or a cDNA library as a template. PCR is conducted for any DNA library according to a known method (see, for example, Michael, A.I. et al., PCR Protocols, a Guide to Methods and Applications, Academic Press (1990)) using sense and antisense primers prepared based on the nucleotide sequence of SEQ IDNO:1, thereby the DNA of the present invention can be obtained. As the DNA library used in the above methods, a DNA library having the DNA of the present invention is selected and used. Any DNA library can be used as long as it comprises the DNA of the present invention. A commercially available DNA library may be also used. Alternatively, a cDNA library may be constructed according to a known method (see J. Sambrook et al., Molecular Cloning, a Laboratory Manual, 2nd ed., Cold Spring Harbor Laboratory, New York (1989)) by selecting cells suitable for the construction of the cDNA library from cells having the DNA of the present invention.

And, the DNA of the present invention can be prepared based on the sequence as disclosed herein by a chemical synthetic technique such as a phosphoramidite method and the like.

The recombinant vector including the DNA of the present invention may have any form such as a cyclic form or a linear form. The recombinant vector may have any other nucleotide sequence in addition to the entire or a part of the DNA of the present invention, if necessary. "A part" means, for example, a DNA encoding a partial peptide of the protein of the present invention. The other nucleotide sequence includes an enhancer sequence, a promoter sequence, a ribosome binding sequence, a nucleotide sequence used for amplifying the number of copies, a nucleotide sequence encoding a signal peptide, a nucleotide sequence encoding other polypeptide, a polyA addition sequence, a splicing sequence, a replication origin, a nucleotide sequence of the gene acting as a selective marker and the like. One preferable example of the recombinant vector of the present invention is an expression vector.

In the gene recombination, it is possible to add a translational initiation codon or a translational stop codon to the DNA of the present invention using a suitable synthetic DNA adaptor, and to newly produce or delete a suitable restriction site within the nucleotide sequence. This is the technique routinely conducted by those skilled in the art. Such a processing can be suitably and easily conducted based on the DNA of the present invention.

As the vector including the DNA of the present invention, a suitable vector is selected and used depending on the type of a host used. The vector may be a plasmid. Alternatively, various viruses may be used, non-limiting examples of which include bacteriophage, baculovirus, retrovirus, vaccinia virus and the like.

The gene of the present invention can be expressed under the control of a promoter sequence inherent in said gene. Using the expression system, an agent promoting or inhibiting the transcription of the gene of the present invention can be efficiently searched. Any other suitable expression promoter can be used by linking it to the promoter sequence inherent in said gene upstream of the gene of the present invention or replacing it with the promoter sequence. In this case, the promoter may be suitably selected depending on a host or an object of expression. For example, if a host is E. coli, a T7 promoter, a lac promoter, a trp promoter, a λPL promoter or the like can be used. If a host is a yeast, a PHO5 promoter, a GAP promoter, an ADH promoter or the like can be used. If a host is an animal cell, a promoter from SV 40, a retro virus promoter or the like can be used. These lists are not exclusive.

A method for introducing the DNA into a vector is known (see J. Sambrook et al., Molecular Cloning, a Laboratory Manual, 2nd ed., Cold Spring Harbor Laboratory, New York (1989)). That is, each of the DNA and the vector is digested with a suitable restriction enzyme and the resultant fragments are ligated with a DNA ligase.

<Protein>

The protein of the present invention can be prepared from various cells and tissues expressing said protein. Alternatively, it can be chemically synthesized in a peptide synthesizer (for example, Peptide Synthesizer Model 433A; Applied Biosystems Japan) or it can be produced by recombination method using a suitable host cell selected from prokaryotic cells and eukaryotic cells. However, a genetic engineering technique and a recombinant protein produced thereby are preferable in view of purity.

A host cell to be transformed with the recombinant vector described in the previous section is not limitative. Many cells of low organisms available in genetic engineering techniques, typical examples of which are E. coli, B. subtilis and S.cerevisiae; and animal cells, typical examples of which are insect cell, COS7 cell, CHO cell and HeLa cell, can be used in the present invention.

The transformant of the present invention can be obtained by transforming a suitable host cell with the recombinant vector of the present invention. As the method of introducing the recombinant vector described in the previous section into a host cell, some methods are known such as an electroporation, a protoplast method, an alkali metal method, a calcium phosphate precipitation method, a DEAE dextran method, a microinjection method, a method using virus particles and the like (see "Handbook of Genetic Engineering", Special Issue of Experimental Medicines, published by Yodosha Co., Ltd. (March 20, 1991)). Either method may be used.

For preparing the present protein by a genetic engineering technique, the above transformant is cultured to obtain a culture mixture followed by purifying the protein. The transformant can be cultured according to a standard method. Many textbooks are available, for example, "Experimental Procedures in Microbiology", edited by The Japanese Biochemical Society, published by Tokyo Kagaku Dozin Co., Ltd. (1992)) describing the culture of transformants, for reference.

As a method for purifying the protein of the present invention from the culture mixture, a suitable method is selected among conventional methods for purifying proteins. The conventional methods include salting out, ultrafiltration, isoelectric precipitation, gel filtration, electrophoresis, various affinity chromatographies including ion-exchange chromatography, hydrophobic chromatography and antibody chromatography, chromatofocusing, adsorption chromatography, reverse phase chromatography and the like. If necessary, HPLC systems or the like may be used to conduct several methods in a suitable order.

It is possible to express the protein of the present invention as a fused protein with any other protein or tag such as glutathione S transferase, Protein A, hexahistidine tag, FLAG tag and the like. The thus-expressed fused protein may be separated with a suitable protease such as thrombin, enterokinase and the like. This may be more effective for the preparation of the protein. For purifying the protein of the present invention, conventional methods may be suitably combined. Especially if the protein is expressed in the form of a fused protein, it is preferable to purify according to a method characteristic to such a form.

One of methods for preparing the present protein by a genetic engineering technique is the synthesis of a cell-free system using a recombinant DNA molecule (J. Sambrook et al., Molecular Cloning, 2nd ed. (1989)).

As mentioned above, the protein of the present invention can be prepared in the form of a single protein or a fused protein with any other different protein. The form of the protein of the present invention is not limited to them. Further, it is possible to transform the protein of the present invention to various forms. For example, it is thought that the protein can be processed according to various methods known for those skilled in the art such as various chemical modifications on the protein, the binding of the protein to a polymeric substance such as polyethylene glycol and the like, the binding of the protein to an insoluble carrier and the like. And, the presence or absence of glycosylation or the difference in glycosylation degree is found depending on a host cell used. It should be understood that all of the above proteins are included within the scope of the present invention as long as said proteins are proteins functions as a scavenger receptor-like protein.

The protein of the present invention can be used as an antigen for the preparation of an antibody and in screening an agent capable of binding to said protein or controlling an activity of said protein and therefore, it is useful.

By culturing the above transformant, especially an animal cell, the ADSE of the present invention can be used for expressing a desired molecule on a surface of the cell. When a suitable fragment of ADSE such as its extracellular region protein fragment is prepared as a soluble protein, a transformant is prepared using a DNA encoding the extracellular region or each domain according to the aforementioned method and cultured so as to secret in a culture supernatant.

When ADSE is present in a periplasm or a cytoplasm of a transformant, the transformant suspended in a suitable buffer is subjected to a treatment such as an ultrasonic treatment, a freeze-thawing treatment, a treatment with lysozyme or the like to destroy a cell wall and/or a cell membrane and further subjected to centrifugation, filtration or the like to obtain a membrane fraction containing the protein of the present invention. Next, this membrane fraction is solubilized with a suitable surfactant to prepare a crude solution, from which the desired protein can be isolated and purified according to the routine method.

<Transgenic animal>

By using the gene adse of the present invention, a transgenic non-human mammalian animal can be produced. The transgenic non-human mammalian animal is also included within the scope of the present invention. The transgenic non-human mammalian animal can be produced according to a routine method conventionally used in the product ion of transgenic animals (see, for example, "Experimental Manual of Genesis, published by Kodansha Scientific Ltd., edited by Motoya KATSUKI under supervision of Tatsuji NOMURA (1987)). That is, the gene or the recombinant vector of the present invention is introduced into a totipotent cell of a non-human animal to produce subjects and thereafter only a subject in which the gene introduced is incorporated in a genome of a somatic cell is selected.

Specifically, in case of a transgenic mouse, a DNA prepared such that the adse gene can be expressed is directly poured into a pronucleic oosperm obtained from a normal C57Black/6 mouse. More specifically, a construct is prepared by introducing the adse gene downstream of a suitable promoter by linking. Thereafter, a linear DNA is obtained by removing the sequence from a prokaryote as much as possible, if necessary. This DNA is directly poured into a pronucleus of the pronucleic oosperm using a fine glass needle.

The oosperm is transplanted in an uterus of another pseudopregnant mouse as an allomother. The pseudopregnant mouse is generally prepared by mating an ICR female mouse with a vasectomized or vasoligated male mouse. A genomic DNA is extracted from a tissue from the transplated embryo and confirmed whether or not the adse gene is introduced by PCR or southern blotting, thereby a transgenic mouse is obtained.

The so-called "knockout mouse" can be produced based on the nucleotide sequence of adse (or a mouse homologous gene of *adse*). The term "knockout mouse" used herein means a mouse in which an endogenous gene encoding the protein of the present invention is knocked out (inactivated). The knockout mouse can be produced by, for example, apositive-negative selectionmethod via homologous recombination (see, for example, US patent Nos. 5, 464, 764, 5, 487, 992 and 5, 627, 059; Proc. Natl. Acad. Sci. USA, Vol. 86, pp. 8932-8935 (1989); Nature, Vol. 342, pp. 435-438 (1989)). Such a knockout mouse is one embodiment of the present invention.

Recently, the production of clone animals by nuclear transplantation in medium or large animals became possible. In this connection, transgenic and knockout animals have been practically produced using this technique. That is, a somatic cell or a germinal cell is subjected to homologous recombination based on the nucleotide sequence of adse (or a homologous gene of adse in each animal) in the same way as that applied to ES cells and then a nucleus is obtained from the resultant cell and used to obtain a clone animal. This animal is a knockout animal in which adse (or a homologous gene of adse in each animal) is lost. Alternatively, adse (or a homologous gene of adse in each animal)is introduced in any cell of any animal and then the resultant nucleus is used to obtain a clone animal, thereby a transgenic animal can be produced. Such a knockout non-human animal and a transgenic non-human animal are one embodiment of the present invention irrespective of its species.

<Antibody>

The antibody of the present invention may be polyclonal or monoclonal. Either antibody can be obtained by referring to a known method (see, for example, "Experimental Procedures in Immunology", edited by Japan Society for Immunology, published by Japan Society for Immunology), as describe below in brief.

For obtaining the novel antibody, an animal is inoculated with the protein of the present invention as an immunizing antigen and if necessary a suitable adjuvant such as Freund's complete adjuvant (FCA), Freund's incomplete adjuvant (FIA) and the like. If necessary, a booster at an interval of 2 to 4 weeks may be conducted. After the booster, blood sample is collected for the preparation of an anti-serum. The protein of the present invention used as an antigen is that obtained in any method as long as it has a purity sufficient to be usable in the preparation of an antibody. A partial polypeptide of the protein of the present invention maybe suitably used as an immunizing antigen. If the polypeptide used as an immunizing antigen is a low-molecular weight polypeptide, i.e. a polypeptide comprising about 10 to 20 amino acids, it may be linked to a carrier such as keyhole limpet hemocyanin (KLH) and the like and used as an antigen. Animals to be immunized include those conventionally used in immunological experiments by those skilled in the art such as rat, mouse, rabbit, sheep, horse, chicken, goat, pig, cattle and the like, among which preferably a species capable of producing the desired antibody is selected and used. However, it is not limited thereto.

A polyclonal antibody can be obtained by purifying the resultant anti-serum. The purification may be conducted by suitably combining known methods such as salting-out, ion exchange chromatography, affinity chromatography and the like.

A monoclonal antibody is obtained as follows: An antibody-producing cell such as a spleen cell, a lymphocyte and the like is taken from an immunized animal. The cell is fused with a myeloma cell strain or the like according to a known method using polyethylene glycol, Sendai virus, an electric pulse or the like to produce a hybridoma. Thereafter, a clone producing an antibody which binds to the protein of the present invention is selected and cultured. By purifying a supernatant of the culture of the selected clone, a monoclonal antibody is obtained. The purification may be conducted by suitably combining known methods such as salting-out, ion exchange chromatography, affinity chromatography and the like.

And, the novel antibody is also obtained by a genetic engineering technique. For example, a mRNA is obtained from a spleen cell or a lymphocyte of an animal immunized with the protein of the present invention or its partial polypeptide or from a hybridoma producing a monoclonal antibody against the protein of the present invention or its partial polypeptide. Based on the thus-obtained mRNA, a cDNA library is constructed. A clone producing the antibody which reacts with the antigen is screened and the thus-screened clone is cultured. The desired antibody can be purified from the culture mixture by combined known methods. When the antibody is used for therapy, a humanized antibody is preferable with respect to immunogenicity. The humanized antibody can be prepared by immunizing a mouse whose immune system has replaced with a human immune system (see, for example, Nat. Genet., Vol. 15, pp. 146-157 (1997)). Alternatively, the humanized antibody can be engineered using hypervariable regions of the monoclonal antibody (Method in Enzymology, Vol. 203, pp. 99-121 (1999)).

<Antisense nucleic acid>

The antisense nucleic acid can be prepared according to a known method (see, for example, edited by Stanley T. Crooke and Bernald Lebleu, in Antisense Research and Applications, published by CRC Publisher, Florida (1993)). If DNA and RNA are native, the antisense nucleic acid of the present invention can be obtained by synthesizing in a chemical synthesizer or conducting PCR using ADSE as a template. Alternatively, a part of derivatives such as methyl phosphonate type and phosphorothioate type can be synthesized in a chemical synthesizer (for example, Expedite Model 8909; Applied Biosystems Japan). Then, such a derivative may be synthesized according to a manual attached to the chemical synthesizer and the thus-synthesized product may be purified by HPLC using a reverse phase chromatography or the like, thereby the antisense nucleic acid can be obtained.

When the DNA and the antisense nucleic acid of the present invention is used as a diagnostic probe, they are labeled with a radioisotope, an enzyme, a fluorescent substance, a luminescent substance or the like according to a known method. Subsequently, a DNA or a mRNA is prepared from a specimen according to a known method and it is used as a test substance. This test substance is reacted with the labeled probe and then the reaction is washed to remove the labeled probe unreacted. If the test substance contains the gene adse or RNA, said antisense nucleic acid binds thereto. The presence or absence of the binding formation can be known by using a luminescence, a fluorescent, a radioactivity or the like from the enzyme, a fluorescent substance or a luminescent substance labeled; or a radioisotope as an index.

When the DNA, the antisense nucleic acid or the recombinant vector of the present invention is used in clinical applications, it is preferable to use those having a purity suitable for the use of a medicine according to any pharmaceutically acceptable method.

The DNA, the antisense nucleic acid or the recombinant vector of the present invention may be used by directly dissolving or suspending in a suitable solvent. Alternatively, it may be used after encapsulating in a liposome or incorporating in a suitable vector. If necessary, it may be used in a suitable dosage form such as injections, tablets, capsules, eye drops, creams, suppositories, spray, poultices in which pharmaceutically acceptable adjuvants are added. Examples of the pharmaceutically acceptable adjuvants are a solvent, a base, astabilizer, apreservative, asolubilizingagent, anexcipient, a buffer and the like.

When the DNA, the antisense nucleic acid or the recombinant vector of the present invention is used in the above dosage form, its administration method and its dose can be selected depending on the age and the sex of a patient, the type and the severity of the disease. Thus, it maybe administered in an amount suitable to improve pathological conditions by the suitable method selected from oral, inhalation, transdermal, intravaginal, intraarticular, intrarectal, intravenous, local, intramuscular, subcutaneous and intraperitoneal administrations.

<Screening method>

The present invention relates to a method of screening an agent capable of controlling the function or the expression of the protein of the present invention, which comprises using the protein of the present invention, a transformant expressing said protein, the DNA of the present invention, a recombinant vector including said DNA, a transformant produced by transformation with said vector, or a transgenic non-human mammalian animal produced by transforming with the DNA of the present invention.

More specifically, the screening method includes:

(1) a method of evaluating a ligand bindability in the presence /absence of a test substance;

(2) a method of screening an agent capable of controling the expression of the protein of the present invention by comparing an expression level of the protein or the gene of the present invention in the presence /absence of a test substance; and the like. Example of the method (1) is a method comprising determining an activity of binding and incorporating a modified LDL in the presence /absence of a test substance in a system as illustrated in Example 3. Example of the method (2) is a method comprising preparing an expression plasmid by linking a reporter gene such as luciferase or the like to the DNA containing an expression control region, a 5'-untranslated region, a region near a translational initiation site or a part of a translation region of the adse gene and determining an expression amount of the reporter gene under the condition where the gene of the present invention is transcripted or translated in the presence/absence of a test substance so as to confirm a transcriptional promotion activity or a transcriptional inhibitory activity of the test substance. The screening method of the present invention comprises the steps of contacting a test substance with the protein of the present invention, a transformant expressing said protein, the DNA of the present invention, a recombinant vector including said DNA, a transformant produced by transformation with said recombinant vector or a transgenic non-human animal produced by transforming with the gene of the present invention; detecting a difference in an activity of the protein of the present invention or an expression level of the DNA of the present invention between a group with the addition of the test substance and a group without the addition of the test substance; and selecting the test substance showing the difference as an agent capable of controlling an activity of the protein of the present invention or an agent capable of controlling the expression of the DNA of the present invention.

An agent capable of controlling an activity of the protein of the present invention may be an agent capable of either enhancing(agonist) or inhibiting (antagonist) the activity of the ADSE protein. The antagonist is preferable. An agent capable of controlling the expression of the DNA of the present invention may be an agent capable of either promoting or inhibiting the expression of the gene adse. An agent capable of inhibiting the expression is preferable.

For confirming whether a test substance controls an activity of the protein of the present invention or controls inhibits the expression of the DNA of the present invention, a difference in the activity of the protein or the expression level of the DNA is determined between the addition and no addition of a test substance in a system capable of confirming the activity of the protein or a system capable of confirming the expression of the DNA. The expression level of the DNA may be determined on the basis of an expression strength of the gene adse into mRNA or the protein. Instead of the expression level of the adse gene or the ADSE protein per se, an expression level of a reporter gene may be detected. The reporter-assay system means an assay method in which an expression amount of a reporter gene arranged downstream of a transcriptional control region is determined so as to screen an agent affecting the transcriptional control region. Examples of the transcriptional control region include a promoter, an enhancer, a CAAT box, a TATA box and the like generally found in a promoter region. As a reporter gene, a CAT (chloramphenicol acetyl transferase) gene, a luciferase gene, a β-galactosidase gene and the like can be used. The expression control region and the 5'-untranslated region of the gene of the present invention can be obtained according to a known method (see "New Experimental Protocol in Cell Engineering", published by Shojunsha Co., Ltd. (1993)). Having function of inhibiting (or suppressing) or enhancing (or promoting) means that a determined value as to the activity of the protein or the expression level of the DNA is different between a group with the addition of a test substance and a group without the addition of a test substance. For example, the inhibition (or suppression) or the enhancement (or promotion) ratio calculated by the following equation is 10% or higher, preferably 30% or higher, more preferably 50% or higher, even preferably 70% or higher, especially preferably 90% or higher.

inhibition (or suppression) or enhancement (or promotion) ratio

(%) =

[absolute value of (determined value of a group without the addition of a test substance) minus (determined value of a group

with the addition of a test substance)] / (determined value of

a group without the addition of a test substance) * 100

Either inhibition or enhancement is suitably determined depending on the kind of a system capable of confirming an activity of the protein or a system capable of confirming the expression of the DNA. The determined value is the same. For example, if a system capable of confirming an activity of the protein is a system for determining an activity of incorporating a modified LDL as shown in Example 3, a radioactivity can be determined. When the determined value in a group with the addition of a test substance is lower than that in a group without the addition of a test substance, the test substance can be judged to have a function of inhibiting the activity of the ADSE protein. Of course, if values from background and/or noises is contained in a determination system, they should be subtracted.

Since the protein of the present invention is a scavenger receptor-like protein, compounds obtained through the search using the screening method or transgenic animal described above are expected to be effective therapeutic or preventive agents for arteriosclerosis and the like. Non-limiting examples of a test substance include proteins, peptides, oligonucleotides, synthetic compounds, naturally occurring compounds, fermented products, cell extracts, plant extracts, animal tissue extracts and the like. The test substance may be either new or known.

BRIEF DESCRIPTION OF DRAWINGS

Fig. 1 shows domain structures of a scavenger receptor protein belonging to class A. I, II, III and M represent subtypes. A represents a cytoplasmic domain. B represents a transmembrane domain. C represents a spacer domain. D is an α-helicalcoiled-coil domain. E represents a collagen like domain. F represents a C-terminal specific domain.

Fig. 2 shows the results of the alignment between ADSE and the known scavenger receptors.

Fig. 3 shows a homology on amino acid sequence between a human scavenger receptor, class A, subtype I and ADSE. A represents a cytoplasmic domain. B represents a transmembrane domain. C represents a spacer domain.

D is an α-helicalcoiled-coil domain. E represents a collagen like domain. F represents a C-terminal specific domain.

Fig. 4 shows an expression profile of the adse gene in human organs and various cells. PHA indicates human peripheral blood leukocytes cultured in the presence of PHA.

EXAMPLES

The present invention will be described in more detail by referring to the following examples which are not to be construed as limiting the scope of the invention.

Example 1 : Cloning of gene adse

(1) Construction of full length cDNA library according to oligocapping method

A poly(A) $^+$RNA was prepared from a human adipose tissue according to the method of Sambrook et al. (Molecular Cloning. A Laboratory Manual, 2nd edition, Cold Spring Harbor Laboratory, Cold Spring Harbor, NY) using an oligo(dT)-cellulose. Next, 5 to 10 μg of the poly (A) $^+$RNA was reacted with 1.2U of Bacterial Alkaline Phosphatase (hereinafter abbreviated as "BAP") (TaKaRa) in a buffer containing 100mM of Tris-HCl (pH 8.0), 5mM of 2-mercaptoethanol and 100U of RNasin (Promega) at 37°C for 40 minutes so as to dephosphorylate the poly(A) $^+$RNA having no cap structure. Thereafter, the reaction liquid was extracted with a mixture of phenol and chloroform (1:1) twice and the poly(A) $^+$RNA was collected as ethanol precipitates. The thus-collected poly (A) $^+$RNA was treated with 20U of Tobacco acid pyrophosphatase (hereinafter abbreviated as "TAP") (Maruyama and Sugano, Gene, Vol. 138, pp. 171-174 (1994)) in a buffer containing 50mM of sodium acetate (pH 5.5), 1mM of EDTA, 5mM of 2-mercaptoethanol and 100U of RNasin at 37°C for 45 minutes so as to remove the cap structure. Thereafter, the reaction liquid was extracted with a mixture of phenol and chloroform (1:1) twice and subjected to ethanol precipitation to thereby collect a BAP-TAP treated poly(A) $^+$RNA.

2 to 4 μg of the thus-collected poly (A) $^+$RNA was ligated to 0.4 μg of 5'-oligomer (5'-AGC AUC GAG UCG GCC UUG UUG GCC UACUGG-3'). This reaction was conducted with 250Uof RNA ligase (TaKaRa) in a buffer containing 50mM of Tris-HCl (pH 7.5), 5mM of $MgCl_2$, 5mM of 2-mercaptoethanol, 0.5mM of ATP, 25% of PEG8000 and 100U of RNAsin at 20°C for 3 to 16 hours. Thereafter, the oligomer unreacted was removed to synthesize a cDNA. That is, 2 to 4 μg of the oligocapped poly (A) $^+$RNA was mixed with 10pmol of a dT adapter-primer (5'-GCG GCT GAA GAC GGC CTA TGT GGC CTT TTT TTT TTT TTT TTT-3') and the mixture was reacted with Superscript II RNase $H^-$ Reverse Transcriptase (Gibco BRL) in the provider's buffer at 42°C for 1 hour.

The reaction was conducted with 15mM of NaOH at 65°C for 1 hour to remove the RNA as a template and then a cDNAwas amplified. 1 μg of the cDNA synthesized from the oligocapped poly(A) $^+$RNA was mixed with 16pmol of a sense primer 1 (5'-AGC ATC GAG TCG GCC TTG TTG-3') and an antisense primer 1 (5'-GCG GCT GAA GAC GGC CTA TGT-3') and amplified using XL PCR kit (Perkin-Elmer). The reaction condition for PCR was 5 to 10 cycles, each cycle comprising heating at 94°C for 1 minute, at 58°C for 1 minute and at 72°C for 10 minutes. The PCR product was extracted with a mixture of phenol and chloroform (1:1) and collected as ethanol precipitates. Thereafter, the thus-collected product was digested with SfiI and subjected to electrophoresis on agarose gel to separate a cDNA of 1,000 bp or higher. This cDNA was inserted into DraIII site of pME18S-FL3 (GenBank accession No. AB009864) which was an expression vector for mammalian cells. Since this DraIII site of pME18S-FL3 was asymmetrical, the terminal of the cDNA fragment was a SfiI site complementary thereto and therefore the cDNA fragment was inserted unidirectionally.

(2) Sequencing of cDNA clone and analysis of information about its deduced protein

A plasmid was prepared from the cDNA library constructed by the method described in the above (1) by means of a pI-100 robot (KURABO). Each clone was sequenced and the resultant sequence was used as data base. For sequencing, a sequence reaction was conducted using AutoCycle sequencing kit (Amersham Pharmacia) and R. O.B. DNA processor (Amersham Pharmacia) according to the provider's protocol in ALF DNA sequencer (Amersham Pharmacia).

The clone C-ADSE02045 obtained by the method (1) contained a cDNA comprising a nucleotide sequence of 3644 base pairs in full length (SEQ IDNO:3), containing an open reading frame (ORF) comprising a nucleotide sequence of 1485 base pairs represented by SEQ ID NO:1, encoding a novel protein which comprises 495 amino acids represented by SEQ ID NO:2. Thisplasmid C-ADSE02045 was deposited in International Patent Organism Depositary (IPDO) (Tsukuba City, Ibaragi Prefecture, Japan) of National Institute of Advanced Industrial Science and Technology as FERM BP-7867 on February 1, 2001.

For searching the homology of the ORF part contained in C-ADSE02045, BLAST (Altschul SF., J. Mol. Evol.,

Vol. 36, pp. 290-300 (1993); Altschul SF. et al, J. Mol. Biol., Vol. 215, pp. 403-410 (1990)) was conducted to search a local agreement in amino acid sequences. As the result, the homology with a human scavenger receptor class A type I was 42% in the cytoplasmic domain (1 to 59a.a.), 59% in the transmembrane domain (60 to 91 a.a.), 26% in the spacer domain (92 to 126 a.a.), 51% in the α-helicalcoiled-coil domain (127 to 305 a.a.), 56% in the collagen like domain (306 to 384 a.a.) and 70% in the C-terminal specific domain (385 to 495 a.a.), from its N-terminal. The total homology was 43%.

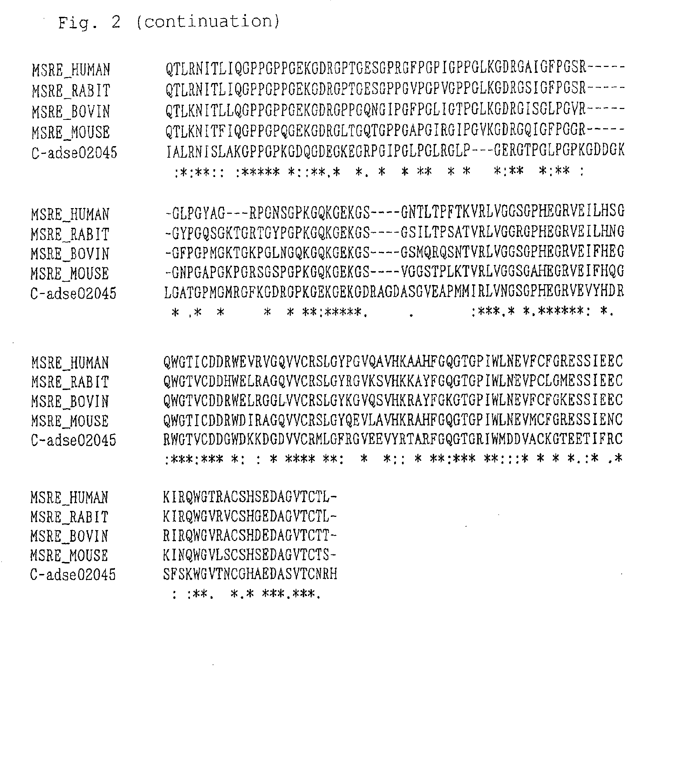

The deduced protein sequence was aligned with an amino acid sequence of SR class A, subtype I from different species obtained by the homology screening using a multiple alignment program Clustal W (Thompson, JD, Higgins, DG, Gibson, TJ., Nucleic Acids Res., Vol. 22, pp. 4673-4680 (1994)). The results are shown in Fig. 2.

Using HMMER (R. Durbin, S. Eddy, A. Krogh, G. Mitchison, Cambridge University Press (1998)) which is a program for screening a homology using a hinded Markov model, pfam (http://www.sanger.ac.uk/Pfam/; E.L.L.Sonnhammer, S.R.Eddy and R. Durbin., Proteins, Vol. 28, p. 405-420 (1997)) of a protein domain database was subjected to screening. As the result, ADSE had a structural homology with MSR types I and II (GenBank Accession No. S08278; Rohrer, L. et al., Nature, Vol. 343, pp. 570-572 (1990)).

Further, a secondary structure of ADSE was analyzed using a computer software PROSITE, COIL, TMpred, PSORT or the like. As the result, domain structures, especially 6 different domain structures found in SR class A, subtype I, i.e. a cytoplasmic domain, a transmembrane domain, a spacer domain, an α-helicalcoiled-coil domain, a collagen like domain and a C-terminal specific domain (from its N-terminal) were all observed.

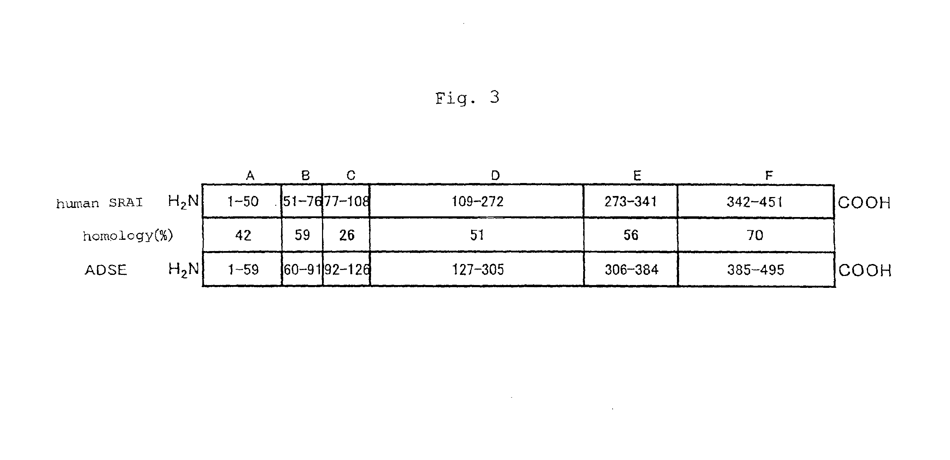

Results of the comparison of the domain structures of ADSE with those of human SR class A, subtype I are shown in Fig. 3.

And, it was confirmed that ADSE had many phosphorylation sites and sugar chain-binding sites, and C-terminal targeting signals of a heme-binding site andmicrobody (peroxisome). This result suggests that ADSE is a protein which is subjected to a posttranslational modification.

Example 2 : Expression in mammalian cells

The expression of a protein was conducted by transfection of the C-ADSE02045 plasmid into COS-1 cells according to the following method.

50 μl of FuGENE6 (Rosche Diagnostics) was mixed with 12.5 μg of the above plasmid according to the provider's protocol and added to COS-1 cells grown in an semiconfluent condition in a 150 $cm^2$ flask. After the cells were cultured in 5% $CO_2$ at 37°C for 72 hours, the cells expressing the ADSE protein was collected. These cells were subjected to an activity assay as shown in Example 3.

Example 3 : Detection of activity of incorporating modified LDL

To the cells expressing the ADSE protein as prepared in Example 2, an oxidized or acetylated LDL was added and then incubated for a predetermined period. As a control group, a transformant produced by transformation with a vector not expressing ADSE was provided and treated similarly. After the incubation, a cellular lipid was extracted with a mixture of hexane and isopropanol (3:2), dried and redissolved in isopropanol. An amount of cholesterol ester in the resultant solution was determined by an enzyme fluorescence method.

Alternatively, an oxidized or acetylated LDL and oleic acid labeled with [$^{14}C$] were added to the cells expressing the ADSE protein and then incubated for a predetermined period. As a control group, a transformant produced by transformation with a vector not expressing ADSE was provided and treated similarly. After the incubation, a cellular lipid was extracted with a mixture of hexane and isopropanol (3:2), dried and redissolved in isopropanol. The resultant solution was subjected to TLC so as to collect cholesterol ester fractions. Their radioactivity was determined (Cell Technology (Supplement), Manuals of Medical Experiments, Series 2 : Strategy of Arteriosclerosis and Hyperlipidemia, p. 148 to 151 and pp. 159 to 164, published by Shunjunsha Co., Ltd.).

As the result, it was found that an incorporated amount of the modified LDL was significantly increased in the group of the cell expressing ADSE protein as compared with that in the control group.

Example 4 Production of knockout mice

(1) Construction of targeting vector

A targeting vector for inactivating (knocking out) an endogeous gene encoding a mouse ADSE protein by homologous recombination (Nikkei Science, pp. 52-62 (May, 1994)) was constructed. A probe for hybridization was prepared according to a routine method by labeling a cDNA of adse with $^{32}P$. Using this probe, a cosmid library (prepared by the same way as that described in "labomanual human genome mapping", editedbyMasaaki HORI and Yusuke NAKAMURA, published by Maruzen Publishing Co., Ltd.) in which a mouse genomic DNA (chromosomal DNA) had introduced was screened to obtain a mouse genomic DNA clone containing an exon encoding a mouse ADSE.

This genomic DNA was linked to a suitable selective marker gene to prepare a plasmid, which was linearlized to thereby obtain a targeting vector.

(2) Introduction of targeting vector to ES cells

Mouse embryonic stem cells (Nature, Vol. 362, pp. 255-258 (1993) and Nature Vol. 326, pp. 292-295 (1987)) cultured in a DEME medium containing 15% fetal bovine serum was treated with trypsin to prepare an unicell. This cell was washed with a phosphate buffer three times to prepare a cell suspension containing $1x10^7$ cells per ml. The above targeting vector in an amount of 25 μg/ml of the cell suspension was added and an electric pulse of 350 V/cm (25 μF) was applied thereto once. Next, $1x10^7$ ES cells were inoculated in a dish (10 cm) and cultured in a maintenance medium for one day and then the maintenance medium was exchanged with a selective medium. Thereafter, the culture was continued while exchanging the medium every two days. After 10 days from the introduction of the targeting vector, several hundreds neomycin-resistant ES cell clones were obtained using a micropipette under a microscope. Each of the resultant clones was cultured in each well of a 24-well plate containing Feeder cells to obtain a replica of neomycin-resistant ES cells.

(3) Screening of knockout ES cells

In order to confirm whether a knockout of an endogenous gene encoding a mouse ADSE by homologous recombination had occurred or not, each of the resultant neomycin-resistant ES cells was determined by a genomic southern blotting. A fragment obtained by digesting a genomic DNA extracted from each of the neomycin-resistant ES cells with EcoRI was subjected to a genomic southern blotting according to a routine method using two probes comprising the sequences of 3' and 5' termini of the mouse genomic DNA, respectively. The purification of DNA was conducted by means of an automated DNA purification robot (Kubota). Thus, it was confirmed that a desired knockout had been occurred in the ES cell clones. This ES cell clones were used in the production of knockout mice as described below.

(4) Production of knockout mice

Each of the ES cell clones obtained in the above in which the endogenous gene encoding the mouse ADSE was inactivated (knocked out) byhomologous recombinationwasmicroinjectedinto blastocytes obtained by mating male and female C57BL6 mice (Nippon Charles River) in a rate of 15 clones per blastocyte. Immediately after the microinjection, about 10 blastocytes per one uterus were transplanted in the uterus of a pseudoparent ICR mouse (Clea Japan Inc.) after 2.5 days from a pseudopregunant treatment. Thus, a knockout chimeric mouse was obtained from each of the ES cell clones. This chimeric mouse was mated with a normal C57BL6 mouse, thereby a mouse with an agouti color brought about from a hair color gene from the ES cells was obtained.

<u>Example 5 : Analysis of expression profile by ATAC-PCR</u>

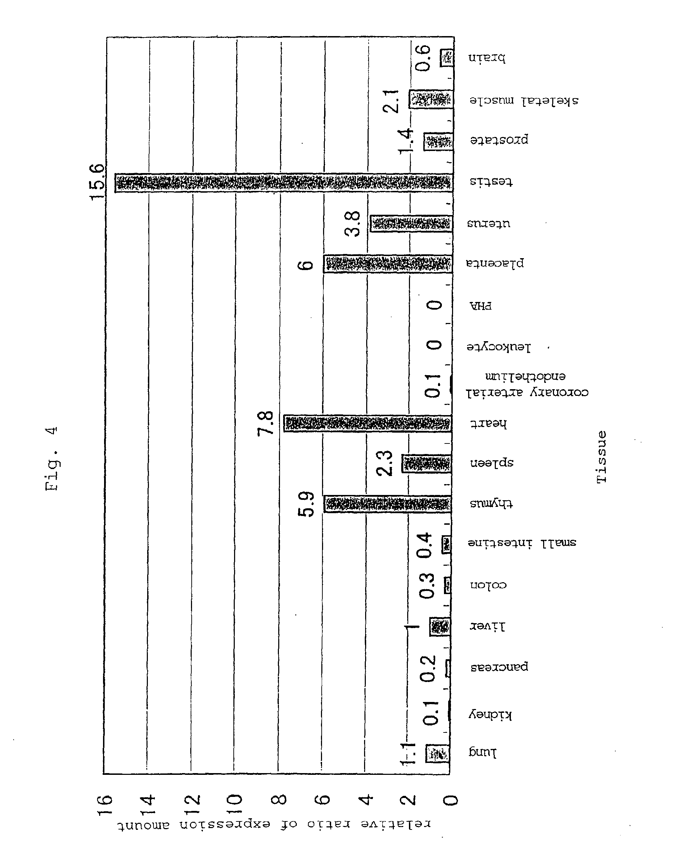

An adaptor-tagged competitive PCR (AEAC-PCR) was conducted according to the method of Kato et al. (Nucleic Acids Research, Vol. 15, pp. 4694-4696 (1997)) by contracting to an outer institute. Ahuman RNA used in this analysis is as follows. A total RNA was prepared from a human coronary arterial endothelial cell (HCAEC) and a human placental tissue according to a routine method. A total RNA was prepared according to a routine method after culturing human peripheral blood leukocytes in the presence of 50 μg/ml of PHA. Total RNAs of human lung, kidney, pancreas, small intestine, thymus, spleen, heart, uterus, testis, prostata and skeletalmusclewerepurchased fromClontech. Total RNAs of liver, colon, leukocyte and brain were purchased from BioChain Institute. Next, a single strand cDNA was synthesized from 6 μg of each of the total RNA according to a routine method using an oligo(dT)-primer. As a reverse transcriptase, Superscript IIRNase $H^-$ Reverse Transcriptase (Invitrogen) was used. A sequence of an adse specific primer used in PCR was 5'-TGT TGC TGT TCC TAT ACC TG-3'. Analytical results are shown in Fig. 4.

<u>EFFECT OF THE INVENTION</u>

ADSE of the present invention is a protein which may develop or progress arteriosclerosis. It is very useful in the development of medicines for preventing or treating the above disease. And, since SR defensively acts against various stresses produced within cells, ADSE is expected to be useful in the development of medicines for pathological conditions and diseases caused by stresses, foreignmatters or modified proteins produced within cells.

The gene adse can be used as antisense medicines and in the gene therapy. The protein ADSE per se or its soluble fragment (extracellular region and each domain) is useful as a soluble protein medicine. Further, an antibody having a reactivity with ADSE or its fragment or a part of said antibody is also useful as an antibody medicine inhibiting ADSE functions *in vivo.*

## Claims

1. The following DNA (a) or (b):

   (a) a DNA comprising the nucleotide sequence represented by SEQ ID NO:1, or
   (b) a DNA hybridizable with the DNA of SEQ ID NO:1 under stringent conditions and encoding a scavenger receptor-like protein.

2. A protein encoded by the DNA as defined in claim 1.

3. The following protein (a) or (b):

   (a) a protein comprising the amino acid sequence represented by SEQ ID NO:2, or
   (b) a scavenger receptor-like protein comprising an amino acid sequence wherein deletion, substitution or addition of one or more amino acids has occurred in the amino acid sequence of SEQ ID NO:2.

4. A recombinant vector including the DNA as defined in claim 1.

5. A transformant produced by transformation with the recombinant vector as defined in claim 4.

6. An antisense nucleic acid inhibiting the expression of the protein as defined in claim 2 or 3.

7. An antisense nucleic acid as defined in claim 6 whose nucleotide sequence is a sequence complementary to the entire or a part of the DNA as defined in claim 1.

8. An antibody against the protein as defined in claim 2 or 3.

9. A method of screening an agent capable of controlling the activity of the protein as defined in claim 2 or 3, which comprises contacting the protein or the transformant expressing the protein with a test substance.

10. A method of screening an agent capable of controlling the expression of the DNA as defined in claim 1, which comprises contacting the vector as defined in claim 4 or the transformant as defined in claim 5 with a test substance.

Fig. 1

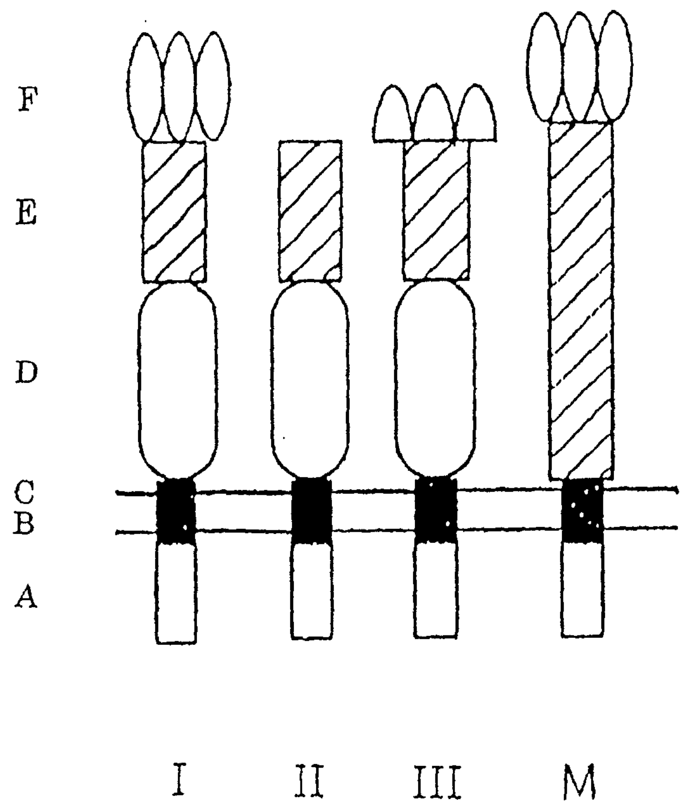

Fig. 2

```
MSRE_HUMAN      -----MEQWDHFHNQQEDTDSCSE-SVKFDARSMTALL-----PPNPKNSP---SLQEKL
MSRE_RABIT      -----MAQWDSFTDQQEDTDSCSE-SVKFDARSNTALL-----PPNPKNGP---PLQEKL
MSRE_BOVIN      -----MAQWDDFPDQQEDTDSCTE-SVKFDARSVTALL-----PPHPKNGP---TLQERM
MSRE_MOUSE      MTKEMTENQRLCPHEREDADCSSE-SVKFDARSMTASL-----PHSTKNGP---SVQEKL
C-adse02045     -----MENKAMYLHTVSDCDTSSICEDSFDGRSLSKLNLCEDGPCHKRRASICCTQLGSL
                     :     . .* * .:  . .**.** :       *   :...   .    :

MSRE_HUMAN      KSFKAALIALYLLVFAVLIPLIGIVAAQL------LKWETKNCSVSSTNANDITQSLTGK
MSRE_RABIT      KSFKAALIALYLLVFAVLIPIIAIMAAQL------LKWEMKNCTVGSINANSVSSSLLGR
MSRE_BOVIN      KSYKTALITLYLIVFVVLVPIIGIVAAQL------LKWETKNCTVGSVNA-DISPSPEGK
MSRE_MOUSE      KSFKAALIALYLLVFAVLIPVVGIVTAQL------LNWEMKNCLVCSRNTSDTSQGPMEK
C-adse02045     SALKHAVLGLYLLVFLILVGIFILAVSRPRSSPDDLKALTRNVNRLNESFRDLQLRLLQA
                .: * *:: ***:** :*: :. : .::       *:   :*    . . .

MSRE_HUMAN      ---GNDSEEEMRFQEVFMEHMSNMEKRIQHILDMEANL--MDTEHFQNFSMT------TD
MSRE_RABIT      ---GNDSEHEVRFREVVMEHISKMEKRIQYISDTEENL--VDSEHFQNFSVT------TD
MSRE_BOVIN      ---GNGSEDEMRFREAVMERMSNMESRIQYLSDNEANL--LDAKNFQNFSIT------TD
MSRE_MOUSE      ---ENTSNVEMRFT-IIMAHMKDMEERIQSISNSKADL--IDTGRFQNFSMA------TD
C-adse02045     PLQADLTEQVWKVQDALQNQSDSLLALAGAVQRLEGALWGLQAQAVQTEQAVALLRDRTG
                    : ::   :.   .  : ..:       :   :  *  :::  .*. . .     *.

MSRE_HUMAN      QRFNDILLQLSTLFSS-------VQGHGNAIDEISKSLISLNTTLLDLQLNIENLNGKIQ
MSRE_RABIT      QRFADVLLQLSTLVPT-------VQGHGNAVDEITRSLISLNTTLLDLHLYVETLNVKFQ
MSRE_BOVIN      QRFNDVLFQLNSLLSS-------IQEHENIIGDISKSLVGLNTTVLDLQFSIETLNGRVQ
MSRE_MOUSE      QRLNDILLQLNSLILS-------VQEHGNSLDAISKSLQSLNMTLLDVQLHTETLHVRVR
C-adse02045     QQSDTAQLELYQLQVESNSSQLLLRRHAGLLDGLARRVGILGEELADVGGVLRGLNHSLS
                *:      ::*  *         :: * . :. ::: :  *.  : *:    . *:  .

MSRE_HUMAN      ENTFKQQEEISKLEERVYNVSAEIMAMKEEQVHLEQEIKGEVKVLNNITNDLRLKDWEHS
MSRE_RABIT      ENTLKGQEEISKLKERVHNASAEIMSMKEEQVHLEQEIKREVKVLNNITNDLRLKDWEHS
MSRE_BOVIN      ENAFKQQEEMRKLEERIYNASAEIKSLDEKQVYLEQEIKGEMKLLNNITNDLRLKDWEHS
MSRE_MOUSE      ESTAKQQEDISKLEERVYKVSAEVQSVKEEQAHVEQEVKQEVRVLNNITNDLRLKDWEHS
C-adse02045     YDVALHRTRLQDLRVLVSNASEDTRRLRLAHVGMELQLKQELAMLNAVTEDLRLKDWEHS
                 ..   :  : .*.  : :.* :   :    :. :* ::* *: :** :*:*********
```

Fig. 2 (continuation)

```
MSRE_HUMAN      QTLRNITLIQGPPGPPGEKGDRGPTGESGPRGFPGPIGPPGLKGDRGAIGFPGSR-----
MSRE_RABIT      QTLRNITLIQGPPGPPGEKGDRGPTGESGPPGVPGPVGPPGLKGDRGSIGFPGSR-----
MSRE_BOVIN      QTLKNITLLQGPPGPPGEKGDRGPPGQNGIPGFPGLIGTPGLKGDRGISGLPGVR-----
MSRE_MOUSE      QTLKNITFIQGPPGPQGEKGDRGLTGQTGPPGAPGIRGIPGVKGDRGQIGFPGGR-----
C-adse02045     IALRNISLAKGPPGPKGDQGDEGKEGRPGIPGLPGLRGLP---GERGTPGLPGPKGDDGK
                 :*:**:: :***** *::**.*  *. *  * **  * *  *:**  *:** :

MSRE_HUMAN      -GLPGYAG---RPGNSGPKGQKGEKGS----GNTLTPFTKVRLVGGSGPHEGRVEILHSG
MSRE_RABIT      -GYPGQSGKTGRTGYPGPKGQKGEKGS----GSILTPSATVRLVGGRGPHEGRVEILHNG
MSRE_BOVIN      -GFPGPMGKTGKPGLNGQKGQKGEKGS----GSMQRQSNTVRLVGGSGPHEGRVEIFHEG
MSRE_MOUSE      -GNPGAPGKPGRSGSPGPKGQKGEKGS----VGGSTPLKTVRLVGGSGAHEGRVEIFHQG
C-adse02045     LGATGPMGMRGFKGDRGPKGEKGEKGDRAGDASGVEAPMMIRLVNGSGPHEGRVEVYHDR
                 * .*  *     *  * **:*****.     .      :***.* *.******: *.

MSRE_HUMAN      QWGTICDDRWEVRVGQVVCRSLGYPGVQAVHKAAHFGQGTGPIWLNEVFCFGRESSIEEC
MSRE_RABIT      QWGTVCDDHWELRAGQVVCRSLGYRGVKSVHKKAYFGQGTGPIWLNEVPCLGMESSIEEC
MSRE_BOVIN      QWGTVCDDRWELRGGLVVCRSLGYKGVQSVHKRAYFGKGTGPIWLNEVFCFGKESSIEEC
MSRE_MOUSE      QWGTICDDRWDIRAGQVVCRSLGYQEVLAVHKRAHFGQGTGPIWLNEVMCFGRESSIENC
C-adse02045     RWGTVCDDGWDKKDGDVVCRMLGFRGVEEVYRTARFGQGTGRIWMDDVACKGTEETIFRC
                :***:*** *: : * **** **:  *  *:: * **:*** **:::* * * *.:* .*

MSRE_HUMAN      KIRQWGTRACSHSEDAGVTCTL-
MSRE_RABIT      KIRQWGVRVCSHGEDAGVTCTL-
MSRE_BOVIN      RIRQWGVRACSHDEDAGVTCTT-
MSRE_MOUSE      KINQWGVLSCSHSEDAGVTCTS-
C-adse02045     SFSKWGVTNCGHAEDASVTCNRH
                 : :**.  *.* ***.***.
```

Fig. 3

| | | A | B | C | D | E | F | |
|---|---|---|---|---|---|---|---|---|
| human SRAI | $H_2N$ | 1-50 | 51-76 | 77-108 | 109-272 | 273-341 | 342-451 | COOH |
| homology(%) | | 42 | 59 | 26 | 51 | 56 | 70 | |
| ADSE | $H_2N$ | 1-59 | 60-91 | 92-126 | 127-305 | 306-384 | 385-495 | COOH |

Fig. 4

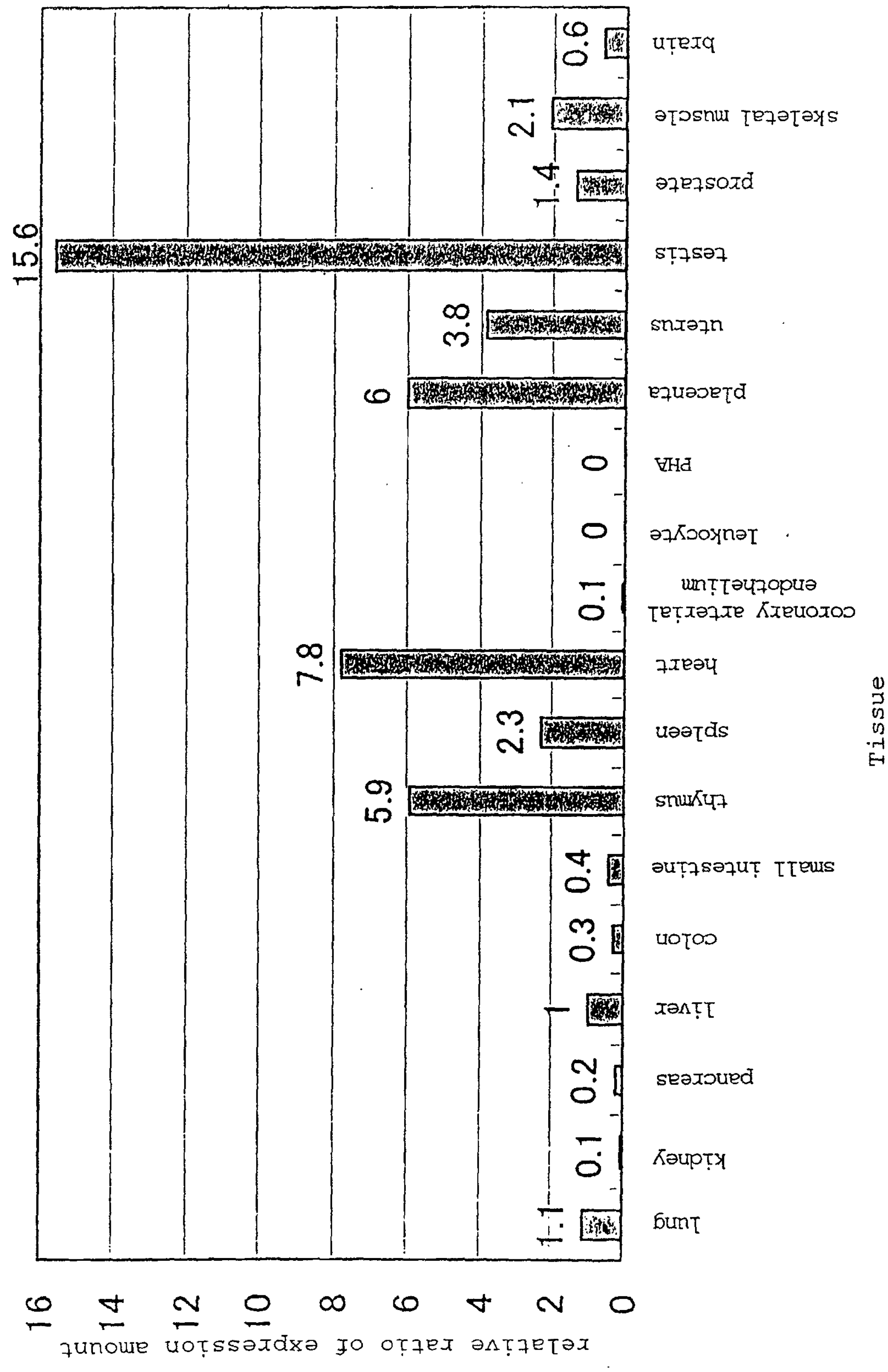
relative ratio of expression amount
0
2
4
6
8
10
12
14
16
Tissue
lung
kidney
pancreas
liver
colon
small intestine
thymus
spleen
heart
coronary arterial endothelium
leukocyte
PHA
placenta
uterus
testis
prostate
skeletal muscle
brain
1.1
0.1
0.2
1
0.3
0.4
5.9
2.3
7.8
0.1
0
0
6
3.8
15.6
1.4
2.1
0.6

## INTERNATIONAL SEARCH REPORT

International application No.
PCT/JP02/01320

A. CLASSIFICATION OF SUBJECT MATTER
Int.Cl[7] C12N15/09, C07K14/705, C07K16/28, C12N5/10, C12Q1/02, C12Q1/68, C12P21/08, G01N33/15, G01N33/50

According to International Patent Classification (IPC) or to both national classification and IPC

B. FIELDS SEARCHED
Minimum documentation searched (classification system followed by classification symbols)
Int.Cl[7] C12N15/09, C07K14/705, C07K16/28, C12N5/10, C12Q1/02, C12Q1/68, C12P21/08

Documentation searched other than minimum documentation to the extent that such documents are included in the fields searched

Electronic data base consulted during the international search (name of data base and, where practicable, search terms used)
REGISTRY(STN), CA(STN), MEDLINE(STN), WPI(DIALOG), BIOSIS(DIALOG), JICST FILE(JOIS), GeneBank/EMBL/DDBJ/GeneSeq, SwissProt/PIR/GeneSeq

C. DOCUMENTS CONSIDERED TO BE RELEVANT

| Category* | Citation of document, with indication, where appropriate, of the relevant passages | Relevant to claim No. |
|---|---|---|
| P,X | WO 01/57260 A1 (Hyseq Inc.), 09 August, 2001 (09.08.01), Full text & AU 200133293 A | 1-10 |
| X | WO 01/07611 A2 (Genentech Inc.), 01 February, 2001 (01.02.01), Claims; sequence No. 398 & AU 200061170 A | 6-8 |
| X | WO 98/45435 A2 (Genetics Inst Inc.), 15 October, 1998 (15.10.98), Claims; sequence No. 1227 & AU 9869566 A & EP 973898 A2 & JP 2001-519666 A | 6-8 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 20 May, 2002 (20.05.02) | 04 June, 2002 (04.06.02) |
| Name and mailing address of the ISA/ Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP02/01320

C (Continuation). DOCUMENTS CONSIDERED TO BE RELEVANT

| Category* | Citation of document, with indication, where appropriate, of the relevant passages | Relevant to claim No. |
|---|---|---|
| A | WO 99/02736 A2 (Millennium Pharm Inc.), 21 January, 1999 (21.01.99), Full text & AU 9883938 A | 1-10 |
| A | EP 808899 A2 (SmithKline Beecham Corp.), 26 November, 1997 (26.11.97), Full text & JP 10-084977 A & US 5916766 A | 1-10 |
| A | WO 92/14482 A1 (Massachusetts Inst Technology), 03 September, 1992 (03.09.92), Full text & EP 572541 A1 & JP 6-508604 A | 1-10 |

Form PCT/ISA/210 (continuation of second sheet) (July 1998)

SEQUENCE LISTING

<110> Mochida Pharmaceutical Co., Ltd.
<120> Novel Scavenger receptor class A
<130> SAP-684-PCT
<160> 3
<170> PatentIn Ver. 2.1

<210> 1
<211> 1485
<212> DNA
<213> *Homo sapiens*

<400> 1
```
atggagaaca aagctatgta cctacacacc gtcagcgact gtgacaccag ctccatctgt     60
gaggattcct ttgatggcag gagcctgtcc aagctgaacc tgtgtgagga tggtccatgt    120
cacaaacggc gggcaagcat ctgctgtacc cagctggggt ccctgtcggc cctgaagcat    180
gctgtcctgg ggctctacct gctggtcttc ctgattcttg tgggcatctt catcttagca    240
gtgtccaggc cgcgcagctc ccctgacgac ctgaaggccc tgactcgcaa tgtgaaccgg    300
ctgaatgaga gcttccggga cttgcagctg cggctgctgc aggctccgct gcaagcggac    360
ctgacggagc aggtgtggaa ggtgcaggac gcgctgcaga accagtcaga ctcgttgctg    420
gcgctggcgg gcgcagtgca gcggctggag ggcgcgctgt ggggctgca ggcgcaggcg    480
gtgcagaccg agcaggcggt ggccctgctg cgggaccgca cgggccagca gagcgacacg    540
gcgcagctgg agctctacca gctgcaggtg gagagcaaca gtagccagct gctgctgagg    600
cgccacgcgg gcctgctgga cgggctggcg cgcagggtgg gcatcctggg cgaggagctg    660
gccgacgtgg gcggcgtgct gcgcggcctc aaccacagcc tgtcctacga cgtggccctc    720
caccgcacgc ggctgcagga cctgcgggtg ctggtgagca acgccagcga ggacacgcgc    780
cgcctgcgcc tggcgcacgt aggcatggag ctgcagctga agcaggagct ggccatgctc    840
aacgcggtca ccgaggacct gcgcctcaag gactgggagc actccatcgc actgcggaac    900
atctccctcg cgaaagggcc accgggaccc aaaggtgatc agggggatga aggaaaggaa    960
ggcaggcctg gcatccctgg attgcctgga cttcgaggtc tgcccgggga gagaggtacc   1020
ccaggattgc ccgggcccaa gggcgatgat gggaagctgg gggccacagg accaatgggc   1080
atgcgtgggt tcaaaggtga ccgaggccca aaaggagaga aaggagagaa aggagacaga   1140
gctggggatg ccagtggcgt ggaggccccg atgatgatcc gcctggtgaa tggctcaggt   1200
ccgcacgagg gccgcgtgga agtgtaccac gaccggcgct ggggcaccgt gtgtgacgac   1260
ggctgggaca agaaggacgg agacgtggtg tgccgcatgc tcggcttccg cggtgtggag   1320
```

gaggtgtacc gcacagctcg attcgggcaa ggcactggga ggatctggat ggatgacgtt 1380
gcctgcaagg gcacagagga aaccattttc cgctgcagct tctccaaatg gggggtgaca 1440
aactgtggac atgccgaaga tgccagcgtg acatgcaaca gacac 1485

<210> 2
<211> 495
<212> PRT

<400> 2

Met Glu Asn Lys Ala Met Tyr Leu His Thr Val Ser Asp Cys Asp Thr
1 5 10 15
Ser Ser Ile Cys Glu Asp Ser Phe Asp Gly Arg Ser Leu Ser Lys Leu
20 25 30
Asn Leu Cys Glu Asp Gly Pro Cys His Lys Arg Arg Ala Ser Ile Cys
35 40 45
Cys Thr Gln Leu Gly Ser Leu Ser Ala Leu Lys His Ala Val Leu Gly
50 55 60
Leu Tyr Leu Leu Val Phe Leu Ile Leu Val Gly Ile Phe Ile Leu Ala
65 70 75 80
Val Ser Arg Pro Arg Ser Ser Pro Asp Asp Leu Lys Ala Leu Thr Arg
85 90 95
Asn Val Asn Arg Leu Asn Glu Ser Phe Arg Asp Leu Gln Leu Arg Leu
100 105 110
Leu Gln Ala Pro Leu Gln Ala Asp Leu Thr Glu Gln Val Trp Lys Val
115 120 125
Gln Asp Ala Leu Gln Asn Gln Ser Asp Ser Leu Leu Ala Leu Ala Gly
130 135 140
Ala Val Gln Arg Leu Glu Gly Ala Leu Trp Gly Leu Gln Ala Gln Ala
145 150 155 160
Val Gln Thr Glu Gln Ala Val Ala Leu Leu Arg Asp Arg Thr Gly Gln
165 170 175
Gln Ser Asp Thr Ala Gln Leu Glu Leu Tyr Gln Leu Gln Val Glu Ser
180 185 190
Asn Ser Ser Gln Leu Leu Leu Arg Arg His Ala Gly Leu Leu Asp Gly
195 200 205

Leu Ala Arg Arg Val Gly Ile Leu Gly Glu Glu Leu Ala Asp Val Gly
210 215 220
Gly Val Leu Arg Gly Leu Asn His Ser Leu Ser Tyr Asp Val Ala Leu
225 230 235 240
His Arg Thr Arg Leu Gln Asp Leu Arg Val Leu Val Ser Asn Ala Ser
245 250 255
Glu Asp Thr Arg Arg Leu Arg Leu Ala His Val Gly Met Glu Leu Gln
260 265 270
Leu Lys Gln Glu Leu Ala Met Leu Asn Ala Val Thr Glu Asp Leu Arg
275 280 285
Leu Lys Asp Trp Glu His Ser Ile Ala Leu Arg Asn Ile Ser Leu Ala
290 295 300
Lys Gly Pro Pro Gly Pro Lys Gly Asp Gln Gly Asp Glu Gly Lys Glu
305 310 315 320
Gly Arg Pro Gly Ile Pro Gly Leu Pro Gly Leu Arg Gly Leu Pro Gly
325 330 335
Glu Arg Gly Thr Pro Gly Leu Pro Gly Pro Lys Gly Asp Asp Gly Lys
340 345 350
Leu Gly Ala Thr Gly Pro Met Gly Met Arg Gly Phe Lys Gly Asp Arg
355 360 365
Gly Pro Lys Gly Glu Lys Gly Glu Lys Gly Asp Arg Ala Gly Asp Ala
370 375 380
Ser Gly Val Glu Ala Pro Met Met Ile Arg Leu Val Asn Gly Ser Gly
385 390 395 400
Pro His Glu Gly Arg Val Glu Val Tyr His Asp Arg Arg Trp Gly Thr
405 410 415
Val Cys Asp Asp Gly Trp Asp Lys Lys Asp Gly Asp Val Val Cys Arg
420 425 430
Met Leu Gly Phe Arg Gly Val Glu Glu Val Tyr Arg Thr Ala Arg Phe
435 440 445
Gly Gln Gly Thr Gly Arg Ile Trp Met Asp Asp Val Ala Cys Lys Gly
450 455 460
Thr Glu Glu Thr Ile Phe Arg Cys Ser Phe Ser Lys Trp Gly Val Thr
465 470 475 480
Asn Cys Gly His Ala Glu Asp Ala Ser Val Thr Cys Asn Arg His
485 490 495

<210> 3
<211> 3644
<212> DNA

<400> 3
atttttata tacggactgg cggcgagagc agctgcagtt cgcatctcag gcagtaccta 60
gaggagctgc cggtgcctcc tcagaacatc tcctgatcgc tacccaggac caggcaccaa 120
ggacagggag tcccaggcgc acaccccca ttctgggtcc cccaggccca gacccccact 180
ctgccacagg ttgcatcttg acctggtcct cctgcaggag tggcccctgt ggtcctgctc 240
tgagactcgt ccctgggcgc ccctgcagcc cctttctatg actccatctg gatttggctg 300
gctgtgggga cgcggtccga ggggcggcct ggctctcagc gtggtggcag ccagctctct 360
ggccaccatg gcaaatgctg agatctgagg ggacaaggct ctacagcctc agccaggggc 420
actcagctgt tgcagggtgt gatggagaac aaagctatgt acctacacac cgtcagcgac 480
tgtgacacca gctccatctg tgaggattcc tttgatggca ggagcctgtc caagctgaac 540
ctgtgtgagg atggtccatg tcacaaacgg cgggcaagca tctgctgtac ccagctgggg 600
tccctgtcgg ccctgaagca tgctgtcctg ggctctacc tgctggtctt cctgattctt 660
gtgggcatct tcatcttagc agtgtccagg ccgcgcagct cccctgacga cctgaaggcc 720
ctgactcgca atgtgaaccg gctgaatgag agcttccggg acttgcagct gcggctgctg 780
caggctccgc tgcaagcgga cctgacggag caggtgtgga aggtgcagga cgcgctgcag 840
aaccagtcag actcgttgct ggcgctggcg ggcgcagtgc agcggctgga gggcgcgctg 900
tgggggctgc aggcgcaggc ggtgcagacc gagcaggcgg tggccctgct gcgggaccgc 960
acgggccagc agagcgacac ggcgcagctg gagctctacc agctgcaggt ggagagcaac 1020
agtagccagc tgctgctgag gcgccacgcg ggcctgctgg acgggctggc gcgcagggtg 1080
ggcatcctgg gcgaggagct ggccgacgtg ggcggcgtgc tgcgcggcct caaccacagc 1140
ctgtcctacg acgtggccct ccaccgcacg cggctgcagg acctgcgggt gctggtgagc 1200
aacgccagcg aggacacgcg ccgcctgcgc ctggcgcacg taggcatgga gctgcagctg 1260
aagcaggagc tggccatgct caacgcggtc accgaggacc tgcgcctcaa ggactgggag 1320
cactccatcg cactgcggaa catctccctc gcgaaagggc caccgggacc caaaggtgat 1380
caggggggatg aaggaaagga aggcaggcct ggcatccctg gattgcctgg acttcgaggt 1440
ctgcccgggg agagaggtac ccccaggattg cccgggccca agggcgatga tgggaagctg 1500
ggggccacag gaccaatggg catgcgtggg ttcaaaggtg accgaggccc aaaaggagag 1560
aaaggagaga aaggagacag agctggggat gccagtggcg tggaggcccc gatgatgatc 1620
cgcctggtga atggctcagg tccgcacgag ggccgcgtgg aagtgtacca cgaccggcgc 1680
tggggcaccg tgtgtgacga cggctgggac aagaaggacg gagacgtggt gtgccgcatg 1740

ctcggcttcc gcggtgtgga ggaggtgtac cgcacagctc gattcgggca aggcactggg 1800
aggatctgga tggatgacgt tgcctgcaag ggcacagagg aaaccatttt ccgctgcagc 1860
ttctccaaat ggggggtgac aaactgtgga catgccgaag atgccagcgt gacatgcaac 1920
agacactgaa agtgggcaga gcccaagttc ggggtcctgc acagagcacc cttcctgcat 1980
ccctggggtg gggcacagct cggggccacc ctgaccatgc ctcgaccaca ccccgtccag 2040
cattctcagt cctcacacct gcatcccagg accgtggggg ccggtcatca tttccctctt 2100
gaacatgtgc tccgaagtat aactctggga cctactgccc gtctctctct tccaccaggt 2160
tcctgcatga ggagccctga tcaactggat caccactttg cccagcctct gaacaccatg 2220
caccaggcct caatatccca gttccctttg gccttttagt tacaggtgaa tgctgagaat 2280
gtgtcagaga caagtgcagc agcagcgatg gttggtagta tagatcattt actcttcaga 2340
caattcccaa acctccatta gtccaagagt ttctacatct tcctccccag caagaggcaa 2400
cgtcaagtga tgaatttccc ccctttactc tgcctctgct ccccatttgc tagtttgagg 2460
aagtgacata gaggagaagc cagctgtagg ggcaagaggg aaatgcaagt cacctgcagg 2520
aatccagcta gatttggaga agggaatgaa actaacattg aatgactacc atggcacgct 2580
aaatagtatc ttgggtgcca aattcatgta tccacttagc tgcattggtc cagggcatgt 2640
cagtctggat acagccttac ctccaggtag cacttaattg gtccattcac ctagactgca 2700
agtaagaaga caaaatgact gagaccgtgt gcccacctga acttattgtc tttacttggc 2760
ctgagctaaa agcttgggtg caggacctgt gtaactagaa agttgcctac ttcagaacct 2820
ccagggcgtg agtgcaaggt caaacatgac tggcttccag gccgaccatc aatgtaggag 2880
gagagctgat gtggagggtg acatggggcc tgcccatgtt aaacctgagt ccagtgctct 2940
ggcattgggc agtcacggtt aaagccaagt catgtgtgtc tcagctgttt ggaggtgatg 3000
attttgcatc ttccaagcct cttcaggtgt gaatctgtgg tcaggaaaac acaagtccta 3060
atggaacccct tagggggaa ggaaatgaag attccctata acctctgggg gtggggagta 3120
ggaataaggg gccttgggcc tccataaatc tgcaatctgc accctcctcc tagagacagg 3180
gagatcgtgt tctgcttttt acatgaggag cagaactggg ccatacacgt gttcaagaac 3240
taggggagct acctggtagc aagtgagtgc agacccacct caccttgggg gaatctcaaa 3300
ctcataggcc tcagatacac gatcacctgt catatcaggt gagcactggc ctgcttgggg 3360
agagacctgg gcacctccag gtataggaac agcaacactc ctggctgaca actaagccaa 3420
tatggcccta ggtcattctt gcttccaata tgcttgccac tccttaaatg tcctaatgat 3480
gagaaactct ctttctgacc aattgctatg tttacataac acgcatgtac tcatgcatcc 3540
cttgccagag cccatatatg tatgcatata taaacatagc actttttact acatagctca 3600
gcacattgca aggtttgcat ttaagttaaa aaaaaaaaaa aaaa 3644